# EUROPEAN PATENT SPECIFICATION

(11) **EP 1 938 808 B1**
(45) Date of publication and mention of the grant of the patent: **12.01.2011**
(21) Application number: 06782879.8
(22) Date of filing: 22.08.2006
(51) Int. Cl.: A61K 9/70, A61K 31/18, A61K 47/02, A61K 47/32

(54) **PREPARATION FOR EXTERNAL USE COMPRISING TAMSULOSIN**
ZUBEREITUNG ZUR TOPISCHEN ANWENDUNG ENTHALTEND TAMSULOSIN
PRÉPARATION À USAGE EXTERNE COMPRENANT DE LA TAMSULOSINE

(30) Priority: 22.08.2005 JP 2005239262
(43) Date of publication of application: 02.07.2008
(73) Proprietor: Hisamitsu Pharmaceutical Co., Inc., Tosu-shi, Saga 841-0017 (JP)
(72) Inventor: KAJITA, Ryoko, Tsukuba-shi, Ibaraki 305-0856 (JP); NOMOTO, Masataka, Tsukuba-shi, Saga 305-0856 (JP)
(74) Representative: Mann, Volker
(86) International application number: PCT/JP2006/316375
(87) International publication number: WO 2007/023791

(56) References cited:
- EP-A- 1 477 541
- WO-A-98/37870
- WO-A-98/37872
- WO-A1-94/16682
- JP-A- 6 256 173
- JP-A- 03 173 816
- JP-A- 04 029 927
- JP-A- 04 368 323
- JP-A- 06 256 173
- JP-A- 2003 210 566
- JP-A- 2003 213 222

## Description

### Technical Field

The present invention relates to a preparation for external use having excellent cohesiveness and pressure-sensitive adhesion.

### Background Art

In recent years, a preparation for percutaneously administering a drug (percutaneous absorption preparation) has been provided for some drugs. There are various forms of the percutaneous absorption preparation, but basically one having a backing and a pressure-sensitive adhesive layer laminated thereon with a drug added to the pressure-sensitive adhesive matrix layer is simple and has excellent adherence to skin. The percutaneous absorption preparation has to be adhered to the surface of a patient's skin so as to follow movement of the skin, but if the preparation is adhered to the skin too strongly, it causes inflammation to the skin when it is peeled off, thus an appropriate pressure-sensitive adhesion is required.

In order for the medicinal efficacy expected by percutaneous application of a drug to be exhibited more quickly and continuously, it is necessary to realize a higher percutaneous absorption of the drug. However, because skin generally functions as a barrier against a penetrant from the environment, it is difficult for the drug to be absorbed via the skin. In order to enhance the percutaneous absorption of a drug, it is necessary to add the drug so that the concentration thereof dissolved in a preparation is close to saturation and, moreover, to use a medium having high affinity for the drug in order to promote diffusion of the drug. However, if these two requirements are satisfied, physical properties such as pressure-sensitive adhesion of the preparation are easily impaired, and particularly for a drug having poor solubility it is difficult to achieve a balance between drug absorption and pressure-sensitive adhesion properties.

Attempts have been made to provide a preparation that gives both excellent drug absorption properties and excellent pressure-sensitive adhesion properties.
For example, there is a tape preparation formed by laminating on a backing a pressure sensitive adhesive containing a basic drug having a free base structure in an acrylic copolymer having a hydroxy group and/or a carboxyl group in the molecule (ref. Patent Publication 1). There is a patch in which a pressure-sensitive adhesive base contains a drug, a plasticizing compound, hydrophilic anhydrous silicic acid, and hydrophobic anhydrous silicic acid, using isopropyl myristate, a polyhydric alcohol fatty acid ester, etc., as the plasticizing compound (ref. Patent Publication 2).

In addition, in the production of a tape-form, etc. crosslinked pressure-sensitive adhesive molding in which a hydroxy group or carboxyl group-containing polymer and a crosslinking agent such as a boron-containing compound are mixed, the crosslinking speed is controlled by crosslinking in the presence of a lower alcohol, and an absorption enhancer such as isopropyl myristate or a sorbitan fatty acid ester is used (ref. Patent Publication 3).
There is a pressure sensitive adhesive composition for medical use such as a patch containing an acrylic copolymer, a plasticizer, and a pseudo-crosslinking agent, in which isopropyl myristate, etc. are used as the plasticizer, and a borate such as ammonium borate is used as the pseudo-crosslinking agent (ref. Patent Publication 4), and there is also a composition such as a patch containing an acrylic copolymer having hydroxyethyl (meth)acrylate as a component, a plasticizer, and a pseudo-crosslinking agent, in which isopropyl myristate, etc. are used as the plasticizer, and boric acid, ammonium borate, etc. are used as the pseudo-crosslinking agent (ref. Patent Publication 5).

Although these techniques aim to relieve skin irritation and give good drug absorption and pressure-sensitive adhesion, there is still the unsolved problem that when a large amount of absorption enhancer or plasticizer is added in order to enhance the drug absorption, the cohesive strength of the pressure-sensitive adhesive matrix layer is degraded, and desired pressure-sensitive adhesion properties cannot be obtained.

Conventional techniques with regard to a percutaneous absorption preparation of a drug having poor solubility, for example, tamsulosin or a salt thereof, are now explained. Tamsulosin hydrochloride has the function of blocking α1 receptors of the urethra and the prostatic portion to thus decrease the internal pressure of the urethra in the prostatic portion, and is commercially available in the form of tablets or capsules as an agent for improving dysuria accompanying prostatic hypertrophy.
For example, there is a preparation for external use such as a patch containing amsulosin or a salt thereof, and an absorption enhancer such as an aqueous alcohol or an aliphatic dicarboxylic acid diester (ref. Patent Publication 6).

Furthermore, there is a percutaneous absorption preparation containing tamsulosin free base that employs isopropyl myristate, etc. as a percutaneous absorption enhancer (ref. Patent Publication 7), and there is also a delivery system for a basic drug containing a laminated composite of a reservoir layer containing a drug and a solubilization improving agent composition, and a backing layer, which employs tamsulosin as the drug and a skin penetration improving agent composition such as an ester component or an acid component (ref. Patent Publication 8).
However, in these conventional techniques, a percutaneous absorption preparation that gives excellent percutaneous absorption for tamsulosin could not be obtained.
Moreover, when a drug is administered, it is preferable to suppress changes in blood concentration of the drug, that is, to reduce the maximum blood drug concentration (peak: P) to minimum blood drug concentration (trough: T) ratio P/T after administration close to 1, since side effects of the drug can be suppressed and the efficacy can be sustained. Such a percutaneous absorption preparation having excellent sustained drug release properties is desired.
WO 98/37872 discloses a pressure-sensitive adhesive comprising tamsulosin and a noncrosslinking acrylate-based pressure sensitive adhesive.

[Patent Publication 1] JP, A, 2-255611
[Patent Publication 2] JP, A, 3-291218
[Patent Publication 3] JP, A, 2003-210566
[Patent Publication 4] JP, A, 2004-35533
[Patent Publication 5] JP, A, 2005-194402
[Patent Publication 6] JP, A, 3-173816
[Patent Publication 7] JP, A, 8-92080
[Patent Publication 8] JP, A, 2000-509734

### Disclosure of Invention

### Problems to be Solved by the Invention

It is therefore an object of the present invention to solve the conventional problems and to provide a preparation for external use that has excellent pressure-sensitive adhesion properties such as cohesive strength even when there are large amounts of components such as a drug, an absorption enhancer, and a plasticizer, and that gives excellent percutaneous absorption when it contains a drug.

### Means for Solving the Problems

While carrying out an intensive investigation in order to solve the above-mentioned problems, the present inventors have found that, a pressure-sensitive adhesive matrix layer formed by adding a boron compound and a silicic acid to a pressure-sensitive adhesive polymer having a hydroxy group in the molecule can, even if a large amount of absorption enhancer, etc. is added, maintain the cohesive strength of the pressure-sensitive adhesive matrix layer and, moreover, when it contains a drug, it can exhibit high absorbability, and as a result of further research, the present invention has been accomplished.

That is, the present invention relates to a preparation for external use that includes a pressure-sensitive adhesive matrix layer, the pressure-sensitive adhesive matrix layer including tamsulosin and/on a pharmaceutically acceptable acid addition salt thereof, a boron compound, a silicic acid, and a pressure-sensitive adhesive base that includes a hydroxy group-containing polymer.
Furthermore, the present invention relates to the preparation for external use, wherein it has a backing.
Moreover, the present invention relates to the preparation for external use, wherein the boron compound is boric acid.
Furthermore, the present invention relates to the preparation for external use, wherein the silicic acid is hydrophilic anhydrous silicic acid.
Moreover, the present invention relates to the preparation for external use, wherein it further includes an absorption enhancer and/or a plasticizer.
Furthermore, the present invention relates to the preparation for external use, wherein the absorption enhancer is one or more selected from the group consisting of a fatty acid, a fatty acid salt, a fatty acid ester, and a fatty acid amide.
Moreover, the present invention relates to the preparation for external use, wherein the absorption enhancer is one or more selected from the group consisting of acetic acid, capric acid, sodium acetate, isopropyl myristate, sorbitan monooleate, sorbitan monolaurate, and lauric acid diethanolamide.
In a further aspect, the preparation for external use gives a maximum blood drug concentration/minimum blood drug concentration ratio after being administered once a day continuously of 1.0 to 1.2.
In a further aspect, the preparation for external use gives a maximum blood drug concentration/minimum blood drug concentration ratio after being administered repeatedly with one day administration and one day suspension of the drug of 1.0 to 1.3.
In a further aspect, the preparation for external use gives a maximum blood drug concentration/minimum blood drug concentration ratio after being administered once every 3.5 days continuously of 1.0 to 1.3.
In a further aspect, the preparation for external use gives a maximum blood drug concentration/minimum blood drug concentration ratio after being administered once every 7 days continuously of 1.0 to 1.3.

### Effects of the Invention

In accordance with the present invention, even with a large amount of a drug and another component such as an absorption enhancer, a preparation for external use is provided in which the pressure-sensitive adhesive matrix layer has excellent cohesiveness, and the drug absorbability is excellent. Furthermore, the present invention gives excellent cohesive strength for the pressure-sensitive adhesive matrix layer and drug absorbability even when a drug having poor solubility such as tamsulosin or an acid addition salt thereof is used.
Since a patch of the present invention has excellent sustained drug release properties and can reduce the maximum blood drug concentration/minimum blood drug concentration ratio P/T, side effects of the drug can be suppressed and, moreover, since the blood drug concentration can be made to stay more or less constant, the effect can be sustained.

### Brief Description of Drawings

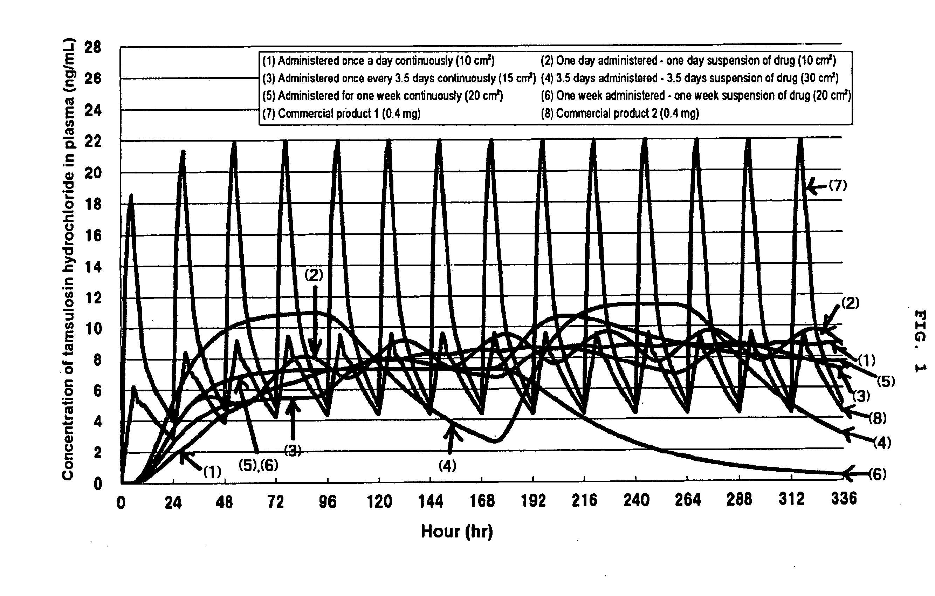

[FIG. 1] Shows a simulation of plasma concentration when patches of the present invention and commercial products are administered.

### Best Mode for Carrying Out the Invention

The preparation for external use of the present invention is constituted from a pressure-sensitive adhesive matrix layer, and the pressure-sensitive adhesive matrix layer contain tamsulosin and/or a pharmaceutically acceptable acid addition salt thereof, a a boron compound, a silicic acid, and a pressure-sensitive adhesive base formed from a polymer having a hydroxy group in the molecule.
It is surmised that the preparation for external use of the present invention is formed by some kind of interaction between the silicic acid and a chemical bond of the hydroxy group of the pressure-sensitive adhesive base with the boron compound, thereby retaining other components contained therein, such as a drug, in the layer shape, thus enhancing cohesive strength.
Each component of the preparation for external use of the present invention is explained in more detail below.

The preparation for external use of the present invention may have a backing, and the pressure-sensitive adhesive matrix layer is laminated on one side of the backing. The backing used here is a sheet-form material that physically supports the pressure-sensitive adhesive matrix layer and protects the pressure-sensitive adhesive matrix layer from the external environment. It is therefore necessary for the backing to have a physical support function in order to handle the preparation by hand or affix it to the skin, and it is not desirable that a component of the pressure-sensitive adhesive matrix layer penetrates into the backing or that the backing is torn when the preparation is peeled off from the skin. As the backing, a film, a fabric, a porous sheet, a paper, or a laminate thereof may be used, and a film is most preferable. With regard to a material of the backing, a polyester such as polyethylene terephthalate or polybutylene terephthalate, a polyolefin such as polyethylene or polypropylene, nylon, rayon, polyurethane, or a metal foil such as aluminum foil may be used, and a polyester is most preferable from the viewpoint of flexibility for following the movement of the skin, and resistance to drug, etc. penetration.

The pressure-sensitive adhesive matrix layer forming the preparation for external use of the present invention is a layered material formed from a mixture that is laminated on one side of the backing, adheres to the skin by having pressure-sensitive adhesion, and supplies a drug contained therein to the skin surface. The pressure-sensitive adhesive matrix layer contains at least tamsulosin and/or a pharmaceutically acceptable acid addition salt thereof, a boron compound, a silicic acid, and a pressure-sensitive adhesive base formed from a polymer having a hydroxy group in the molecule, and may contain as appropriate another component such as a tackifying resin, a plasticizer, a filler, an absorption enhancer, a solubilizing agent, or a stabilizer. The thickness of the pressure-sensitive adhesive matrix layer is preferably in the range of 20 to 200 µm. Such a range can give sufficient cohesive strength for retaining the shape and good pressure-sensitive adhesion to the skin.

The drug contained in the pressure-sensitive adhesive matrix layer forming the preparation for external use of the present invention is the main constitution that is absorbed from the pressure-sensitive adhesive matrix layer into the body via the skin, and exhibits a physiological effect. The drug is tamsulosin and/or a pharmaceutically acceptable acid addition salt thereof.
Excellent effects are exhibited with tamsulosin and pharmaceutically acceptable acid addition salts thereof, which have poor solubility. The pharmaceutically acceptable acid addition salts referred to here include the hydrochloride (formula below), the mesylate, and the citrate, and the hydrochloride is particularly suitably used. 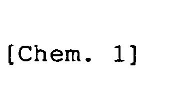 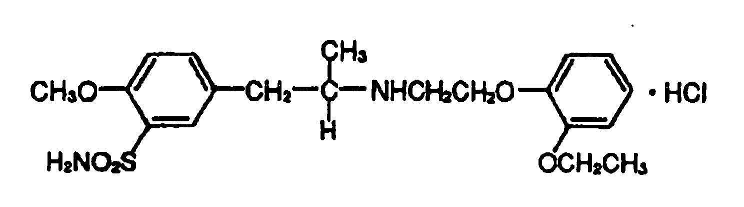 Tamsulosin hydrochloride is an α1 receptor blocker and is prescribed for the purpose of improving dysuria.
The drug is normally preferably added in the range of 1 to 30 mass % relative to the entire pressure-sensitive adhesive matrix layer. The amount thereof added should be determined as appropriate according to an administration plan, but when the therapeutic effect is taken into consideration it is preferably in the range of 1 to 30 mass % relative to the entire pressure-sensitive adhesive matrix layer.
Furthermore, the molecular weight of the drug used is suitably 600 or below when percutaneous absorbability is taken into consideration.

The drug and the pressure-sensitive adhesive base used in the preparation for external use of the present invention are selected as appropriate, mainly based on the relationship between the characteristics of the chemical structure of the drug and a functional group of the pressure-sensitive adhesive base rather than on the physiological effect of the drug. In the preparation for external use of the present invention, if the pressure-sensitive adhesive base has a carboxyl group and the drug is an amine-based drug, hardly any drug is delivered to the body. It appears that this is because the carboxyl group of the pressure-sensitive adhesive base and an amino group of the drug form an ionic bond, thereby greatly suppressing diffusion of the drug in the pressure-sensitive adhesive matrix layer. Therefore, a combination of the pressure-sensitive adhesive base and the drug should be selected so that such an ionic bond is not formed. That is, when the pressure-sensitive adhesive base does not have an acidic dissociable functional group, the drug may be non-ionically dissociable or have a basic dissociable group. When the pressure-sensitive adhesive base does not have an ionic dissociable group, the drug may be non-ionically dissociable or have any dissociable group such as an acid or a base. When the pressure-sensitive adhesive base does not have a basic dissociable functional group, the drug may be non-ionically dissociable or may have an acidic dissociable group. The acidic dissociable functional group referred to above is a carboxyl group, a sulfonic acid group, etc., and examples of the basic dissociable functional group include a primary amino group, a secondary amino group, a tertiary amino group, and a quaternary ammonium group.

The polymer having a hydroxy group in the molecule, which is used as the pressure-sensitive adhesive base of the pressure-sensitive adhesive matrix layer forming the preparation for external use of the present invention, is formed by some type of bond between the hydroxy group, the boron compound, and the silicic acid, although the mechanism is not clear, thereby retaining other components, such as a drug contained in the pressure-sensitive adhesive matrix layer in the layered shape, allows these components to diffuse and move to the skin surface, and provides pressure-sensitive adhesion to the skin. The polymer is preferably a vinyl-based polymer having a hydroxy group in the molecule. The vinyl-based polymer having a hydroxy group in the molecule is preferably a (meth)acrylic acid ester copolymer or vinyl acetate copolymer having a hydroxy group in the molecule, etc.

The (meth)acrylic acid ester copolymer has an acrylic acid ester or methacrylic acid ester as a main monomer, an acrylic acid ester or methacrylic acid ester monomer having a hydroxy group as its crosslinking site is copolymerized, and another monomer may be copolymerized in order to modify the pressure-sensitive adhesion properties, etc. of the base.

Preferred examples of the main monomer of the (meth)acrylic acid ester copolymer include acrylic acid or methacrylic acid methyl, ethyl, propyl, butyl, pentyl, hexyl, heptyl, octyl, nonyl, decyl, undecyl, dodecyl, tridecyl, etc. linear-chain alkyl esters, or 2-ethylhexyl, etc. branched alkyl esters. The proportion of the main monomer is preferably in the range of 30 to 99 mol % relative to the entire copolymer.

The hydroxy group-containing acrylic acid ester or methacrylic acid ester monomer of the(meth)acrylic acid ester copolymer is preferably an acrylic acid ester or methacrylic acid ester such as 2-hydroxyethyl acrylate, 3-hydroxypropyl acrylate, 4-hydroxybutyl acrylate, 2-hydroxyethyl methacrylate, 3-hydroxypropyl methacrylate, or 4-hydroxybutyl methacrylate, vinyl alcohol, allyl alcohol, 3-buten-1-ol, 3-buten-2-ol, etc. The proportion of the hydroxy group-containing monomer is preferably in the range of 1 to 15 mol % relative to the entire copolymer. The hydroxy group forms a bond with the boron compound and the silicic acid and functions as a crosslinking site of the pressure-sensitive adhesive matrix layer. However, it is undesirable to have a large amount of the hydroxy group-containing monomer since it degrades the pressure-sensitive adhesion of the matrix layer.

Examples of other comonomers of the (meth)acrylic acid ester copolymer include vinyl acetate, vinyl propionate, allylamine, styrene, vinylpyrrolidone, methylvinylpyrrolidone, vinylpyridine, vinylpiperidone, vinylpiperazine, vinylpyrazine, and acrylamide.
A monomer having a carboxyl group such as acrylic acid or methacrylic acid is preferably not copolymerized with the (meth)acrylic acid ester copolymer, and since such a monomer tends to inhibit release of the drug from the preparation, if one is added, it should be added at 5% or below.
Examples of the pressure-sensitive adhesive base of the pressure-sensitive adhesive matrix layer of the present invention include commercial pressure sensitive adhesives such as Duro-Tak 87-2287 (manufactured by Nippon NSC Ltd.).
The proportion of the polymer having a hydroxy group in the molecule is preferably at least 25 mass % relative to the entire pressure-sensitive adhesive matrix layer (the same applies below), and in this range sufficient cohesive strength is obtained for the pressure-sensitive adhesive matrix layer.

In addition to the above-mentioned pressure-sensitive adhesive base, in order to modify the pressure-sensitive adhesion properties of the pressure-sensitive adhesive matrix layer or absorption of the drug, an additional pressure-sensitive adhesive base may be added. From the viewpoint of drug absorption, the additional pressure-sensitive adhesive base is preferably a polymer that does not have a dissociable group with a charge that forms a pair with the ionically dissociable charge of the drug. Specific examples thereof include non-ionically dissociable polymers such as polyvinylpyrrolidone, a vinylpyrrolidone copolymer, polyisobutylene, polyisoprene, polyvinyl acetate, a vinyl acetate copolymer such as an ethylene-vinyl acetate copolymer, a styrene-butadiene-styrene block copolymer, a styreneisoprene-styrene block copolymer, and a styrene-butadiene rubber. The proportion of the additional pressure-sensitive adhesive base is preferably no greater than 60 mass % relative to the entire pressure-sensitive adhesive matrix layer, and in such a range the cohesive strength of the pressure-sensitive adhesive matrix layer is not degraded.

The boron compound used in the preparation for external use of the present invention forms an ester bond with the hydroxy group of the pressure-sensitive adhesive base to thus become a crosslinking site of the pressure-sensitive adhesive matrix layer, thereby enhancing the cohesive strength of the pressure-sensitive adhesive matrix layer. As the boron compound, a borate such as sodium tetraborate or ammonium tetraborate or, furthermore, a boron compound having a valence of +3 such as boric acid, methyl borate, ethyl borate, propyl borate, or butyl borate may be employed, and boric acid is preferable. Boric acid is a compound represented by H₃BO₃, and anhydrous boric acid or a hydrate may be used.

In order to uniformly prepare a pressure-sensitive adhesive matrix layer of the preparation for external use of the present invention, boric acid is mixed with another component once it has been dissolved in a solvent, thus achieving uniform crosslinking. The boric acid may be dissolved in water, an alcohol, glycerol, etc. Among the above solvents, methanol or ethanol is preferable.
The amount of boron compound added is preferably in the range of 1 to 8 mass % relative to the entire pressure-sensitive adhesive matrix layer, and in such a range the cohesive strength can be enhanced and sufficient pressure-sensitive adhesion can be obtained for the pressure-sensitive adhesive matrix layer.

The silicic acid used in the preparation for external use of the present invention promotes formation of a bond between the hydroxy group and boric acid, imparts shape retention to the pressure-sensitive adhesive matrix layer by physical bonding between silicic acid particles, and enhances the cohesive strength. Examples of the silicic acid include silica gel produced from sodium silicate, colloidal silica (dispersion), which is ultra-fine particles of anhydrous silicic acid produced from water glass, and ultra-fine particulate anhydrous silica (light anhydrous silicic acid) produced in the gas phase from a chloride of ferrosilicon. Among them, anhydrous silicic acid is preferable to hydrated silicic acid, and light anhydrous silicic acid is more preferable.

The particle size of the silicic acid is preferably small; the average particle size is preferably no greater than 50 µm, and particularly preferably no greater than 16 µm. In this range, there is hardly any coating unevenness when preparing a pressure-sensitive adhesive matrix; material having a small particle size has a large surface area and an excellent effect in enhancing drug absorption, and also has excellent effects in retaining the shape of the pressure-sensitive adhesive matrix layer and enhancing the cohesive strength.

With regard to anhydrous silica particles, those having a hydrophilic surface are preferable in terms of drug absorption, and give good mixing properties with a medium (solvent) when preparing the pressure-sensitive adhesive matrix.
The amount of silicic acid added is preferably in the range of 0.2 to 10 mass % relative to the entire pressure-sensitive adhesive matrix layer, and in this range a liquid to be applied has a good flowability when preparing the pressure-sensitive adhesive matrix, and the pressure-sensitive adhesive matrix thus obtained has a uniform thickness.
The specific surface area of the particles is preferably at least 100 cm²/g, and more preferably at least 300 cm²/g, from the viewpoint of enhancement of the shape retention of the pressure-sensitive adhesive matrix layer.
With regard to the silicic acid, for example, 7 to 16 µm light anhydrous silicic acid is commercially available and preferably used.

Although the reason that adding the silicic acid promotes a crosslinking reaction of the boron compound is not clear, it is surmised that the boron compound is bonded to -OSi=O or - OSiOH of the surface of anhydrous silicic acid to thus increase the electrophilicity of the B atom, thereby making it easy to form a crosslinking bond with the oxygen atom of the OH of the pressure-sensitive adhesive base. It is also surmised that a crosslinking site is formed by an ester exchange reaction that forms a bond between the oxygen atom of the OH of the base polymer and anhydrous silicic acid.
Since the above-mentioned reaction progresses gradually, there is enough time between mixing these compounds and spreading the liquid into a sheet form, thus making it convenient for the production of a patch.
With the above-mentioned compounds, the preparation for external use of the present invention has excellent cohesive strength and appropriate pressure-sensitive adhesive power.

The present invention may further contain a percutaneous absorption enhancer in order to enhance the drug absorption. The above-mentioned compounds enable the preparation for external use of the present invention to maintain the cohesive strength of the pressure-sensitive adhesive matrix layer even when it contains a large amount of another component such as a percutaneous absorption enhancer, thus high drug absorption can be achieved.
The absorption enhancer may be used in order to enhance absorption of the drug. The absorption enhancer that can be used is not particularly limited and is selected from known absorption enhancers according to the drug administration form. Specific examples thereof include lower alcohols, saturated or unsaturated straight-chain or branched aliphatic alcohols, saturated or unsaturated aliphatic ethers, saturated or unsaturated fatty acids, fatty acid salts, fatty acid esters such as a sorbitan fatty acid ester or a fatty acid glycerol ester, fatty acid ester amides, terpenes, vegetable oils or fats such as olive oil, animal oils or fats such as squalene, N-methylpyrrolidone, crotamiton, and azacycloalkane derivatives.
Examples of the saturated or unsaturated fatty acids include organic acids having 2 to 4 carbons such as acetic acid, propionic acid, butyric acid, lactic acid, glycolic acid, malic acid, tartaric acid, and citric acid, and fatty acids such as straight-chain or branched saturated fatty acids having 6 to 18 carbons such as caproic acid, caprylic acid, capric acid, lauric acid, myristic acid, palmitic acid, stearic acid, linoleic acid, and linolenic acid, or unsaturated fatty acids such as oleic acid, linoleic acid, and linolenic acid, and acetic acid, capric acid, lauric acid, etc. are preferably used. Examples of the fatty acid salts include potassium, sodium, calcium, and magnesium salts of the above-mentioned fatty acids; sodium acetate, sodium caprate, sodium laurate, etc. are preferably used, and sodium acetate is particularly preferable. Examples of the fatty acid esters includes esters formed from the above-mentioned fatty acids and, as an alcohol, an aliphatic alcohol such as methanol, ethanol, or propanol, a polyhydric alcohol such as ethylene glycol, propylene glycol, glycerol, or polyethylene glycol, or a sugar alcohol such as sorbitol and, for example, isopropyl myristate, sorbitan monooleate, sorbitan monolaurate, etc. are preferable. With regard to the fatty acid amides, amides between the above-mentioned fatty acids and an amine such as diethanolamine, for example, lauric diethanolamide, are preferable.

When the drug to be administered is tamsulosin or tamsulosin hydrochloride, the absorption enhancer is preferably a fatty acid, a fatty acid salt, a fatty acid ester, or a fatty acid amide. As the fatty acid, acetic acid, capric acid, lauric acid, etc. are preferably used, and acetic acid and capric acid are particularly preferable. As the fatty acid salt, sodium acetate, sodium caprate, sodium laurate, etc. are preferably used, and sodium acetate is particularly preferable. As the fatty acid ester, isopropyl myristate, sorbitan monooleate, and sorbitan monolaurate are preferable. As the fatty acid amide, lauric diethanolamide is preferable. Among them, in the case of tamsulosin, acetic acid is particularly preferable, and in the case of tamsulosin hydrochloride, sodium acetate and capric acid are preferable.
The proportion of the absorption enhancer is preferably in the range of 1 to 30 mass % relative to the entire pressure-sensitive adhesive matrix layer, and in such a range it does not cause any residual adhesive due to degradation of the cohesive strength of the pressure-sensitive adhesive matrix layer.

The pressure-sensitive adhesive matrix layer of the present invention may further contain as an additional component, for example, a tackifying resin, a plasticizer, a filler, a solubilizing agent, a stabilizer, etc.
The tackifying resin is used for enhancing the pressure-sensitive adhesion of the pressure-sensitive adhesive matrix layer. The tackifying resin that can be used is not particularly limited, and is selected from known tackifying resins according to the drug administration mode. Specific examples thereof include an alicyclic saturated hydrocarbon resin, a hydrogenated rosin glycerol ester, an aliphatic hydrocarbon resin, and a terpene resin. The proportion of the tackifying resin is preferably no greater than 40 mass % relative to the entire pressure-sensitive adhesive matrix layer.

The plasticizer may be used in order to modify the pressure-sensitive adhesion properties of the pressure-sensitive adhesive matrix layer, the flow properties of a coating solution in the production thereof, and the drug absorption. By adding a plasticizer, the cohesive strength of the pressure-sensitive adhesive matrix layer is degraded, and flexibility is imparted. The plasticizer that can be used is not particularly limited, and is selected from known plasticizers and softening agents according to the drug administration form. Specific examples thereof include liquid paraffin, liquid polybutene, liquid polyisoprene, castor oil, cottonseed oil, palm oil, and coconut oil. The proportion of the plasticizer is preferably no greater than 40 mass % relative to the entire pressure-sensitive adhesive matrix layer.

The filler may be used mainly for modifying the pressure-sensitive adhesion properties of the pressure-sensitive adhesive matrix layer, or for shielding light. Adding a filler suppresses the pressure-sensitive adhesive power. The filler that can be used is not particularly limited and is selected from known fillers according to the drug administration mode.
Specific examples thereof include metal oxides such as zinc oxide and titanium oxide, metal hydroxides such as aluminum hydroxide, silicate compounds, and calcium carbonate. The proportion of the filler is preferably no greater than 10 mass % relative to the entire pressure-sensitive adhesive matrix layer, and care must be taken with the proportion so that the filler does not inhibit crosslinking of the pressure-sensitive adhesive matrix layer.

The solubilizing agent has the function of enhancing the drug absorption by increasing the solubility of the drug in the pressure-sensitive adhesive matrix layer. By adding a solubilizing agent, the cohesive strength of the pressure-sensitive adhesive matrix layer is often degraded. The solubilizing agent that can be used is not particularly limited, and is selected as appropriate from known solubilizing agents according to the drug.

With regard to the stabilizer, in order to enhance the stability of the pressure-sensitive adhesive matrix layer or the drug, a known antioxidant, UV absorber, etc. may be added as appropriate. Specific examples of the stabilizer include ascorbic acid derivatives, tocopherol derivatives, dibutylhydroxytoluene, an edetate, and 4-tert-butyl-4'-methoxydibenzoylmethane.

A separator used for the preparation for external use of the present invention is laminated so as to cover the pressure-sensitive adhesive matrix in order to protect it during storage and is peeled off when using the preparation for external use. The separator may be a film, a paper, or a laminate thereof, and is not particularly limited, and a film is most preferable. Specific examples of the material of the separator include a polyester such as polyethylene terephthalate, polybutylene terephthalate, or polyethylene naphthalate, a polyolefin such as polyethylene or polypropylene, a nylon, and a metal foil such as an aluminum foil, and from the viewpoint of stability over time of the physical properties of the preparation, a polyester is most preferable. The surface of the separator is preferably subjected to a release treatment such as a silicone treatment in order to facilitate peeling off.

With regard to the preparation for external use of the present invention, for example, the maximum blood drug concentration/minimum blood drug concentration ratio P/T when it is administered once a day continuously is preferably 1.0 to 1.2, the P/T ratio after being administered repeatedly with one day administration and one day suspension of the drug is preferably 1.0 to 1.3, the P/T ratio after being administered once every 3.5 days continuously is preferably 1.0 to 1.3, and the P/T ratio after being administered once every 7 days continuously is preferably 1.0 to 1.3, from the viewpoint of alleviation of side effects of the drug and sustaining the effect. The maximum blood drug concentration and the minimum blood drug concentration are values obtained by measuring the blood drug concentration when the change in drug concentration in the blood attains a fixed pattern while the drug is repeatedly administered under the respective administration conditions, that is, the pattern of change reaches a steady statue.

### Examples

The present invention is further explained below by reference to Examples of the present invention.

### Test 1

### Patch preparation method

(1) Boric acid was added to methanol to give a 10 mass % solution.
(2) 0.40 g of Aerosil (AEROSIL 200) and 2.25 g of ethyl acetate were added to 3.32 g of the solution from (1), and mixed for several minutes to give a uniform dispersion.
(3) 9.7 g of Duro-Tak 87-2287 liquid was further added to the dispersion from (2), and mixed for about 1 hour.
(4) 0.25 g of tamsulosin hydrochloride, 0.07 g of sodium acetate, 0.25 g of isopropyl myristate (IPM), and 0.15 g of sorbitan monolaurate (SML) were mixed for about 15 hours, 11.78 g of the liquid from (3) was further added thereto, and mixing was carried out for about 1 hour to give a uniform coating liquid.
(5) The liquid from (4) was spread over a silicone-treated surface of a separator (polyethylene terephthalate film, 75 µm), and dried at 80°C for 10 minutes. Subsequently, a backing (polyethylene terephthalate film, sand matte treated, 25 µm) was laminated, thus giving a patch of Example 4 of the present invention.

Patches having the mixing proportions below were prepared in the same manner as in Example 4. The mixing proportions are expressed as mass %.

**[Table 1]**

| | Tamsulosin hydrochloride | Duro-Tak 87-2287 | IPM | SML | Sodium acetate | Aerosil | Boric acid | Coating thickness g/m² |
|---|---|---|---|---|---|---|---|---|
| Reference Ex. 1 | 0 | Remainder | 5 | 3 | 0.92 | 6 | 1.8 | 110 |
| Reference Ex. 2 | 0 | Remainder | 5 | 3 | 0.92 | 6 | 3 | 120 |
| Reference Ex. 3 | 0 | Remainder | 5 | 3 | 0.92 | 6 | 5 | 100 |
| Ex. 4 | 5 | Remainder | 5 | 3 | 1.38 | 6 | 5 | 100 |
| Ex. 5 | 5 | Remainder | 8.6 | 3 | 0.92 | 6 | 1.8 | 100 |
| Ex. 6 | 5 | Remainder | 8.6 | 3 | 0.92 | 6 | 1.8 | 60 |
| Ex. 7 | 5 | Remainder | 5 | 3 | 0.92 | 6 | 5 | 100 |
| Ex. 8 | 5 | Remainder | 5 | 3 | 0.92 | 6 | 3 | 100 |
| Ex. 9 | 5 | Remainder | 5 | 3 | 0.92 | 6 | 4 | 100 |
| Comp. Ex. 1 | 0 | Remainder | 9.1 | 3.2 | 1.05 | 0 | 1.9 | 60 |
| Comp. Ex. 2 | 0 | Remainder | 8.6 | 3 | 0.92 | 0 | 1.8 | 60 |
| Comp. Ex. 3 | 0 | Remainder | 9.1 | 3.2 | 1.05 | 1.9 | 0 | 60 |
| Comp. Ex. 4 | 0 | Remainder | 5 | 3 | 0.92 | 0 | 5 | 85 |
| Comp. Ex. 5 | 5 | Remainder | 8.6 | 3 | 0.92 | 0 | 2 | 110 |
| Comp. Ex. 6 | 5 | Remainder | 8.6 | 3 | 0.92 | 2 | 0 | 110 |
| Comp. Ex. 7 | 5 | Remainder | 8.6 | 3 | 0.92 | 0 | 1.8 | 70 |
| Comp. Ex. 8 | 5 | Remainder | 8.6 | 3 | 0.92 | 1.8 | 0 | 65 |
| Comp. Ex. 9 | 5 | Remainder | 8.6 | 3 | 0.92 | 0 | 0 | 60 |
| Comp. Ex. 10 | 5 | Remainder | 5 | 3 | 0.92 | 0 | 1.8 | 140 |
| Comp. Ex. 11 | 5 | Remainder | 5 | 3 | 0.92 | 6 | 0 | 120 |
| Comp. Ex. 12 | 5 | Remainder | 5 | 3 | 0.92 | 0 | 0 | 130 |
| Comp. Ex. 13 | 5 | Remainder | 3 | 3 | 0.92 | 0 | 1.8 | 130 |
| Comp. Ex. 14 | 5 | Remainder | 5 | 3 | 0.92 | 0 | 5 | 90 |

### Tack Test

An appropriate size of a patch was affixed to a 100 g ring-shaped load and set in a probe tack tester in accordance with ASTM D-2979, a stainless steel probe having a diameter of 5 mm was pressed against the pressure-sensitive adhesive surface of the patch for 1 sec at a speed of 5 mm/sec, and the force [gf] required for peeling off the probe at a speed of 5 mm/sec was measured.

### Peel Test

A 1 cm x 5 cm patch was affixed to a stainless steel plate, allowed to stand for 30 minutes, and then set in an Instron type tensile tester, and the force [gf] required for peeling off at 180° at 300 mm/min was measured.

### Skin Permeation Test

The skin of the ventral part of a hairless mouse was exfoliated off, and mounted on a flow through cell with hot water at 37°C circulating around its outer periphery so that the dermal side was on a receptor layer side. Subsequently, a patch with an application area of 5 cm² was administered to the stratum corneum side of the skin, PBS was used as the receptor layer, and PBS was flowed at 4.5 mL/hr as the receptor layer and the receptor layer was sampled every 2 hours up to 24 hours. The flow rate was measured, the drug concentration was measured using high performance liquid chromatography, a drug permeation rate per hour was calculated from the measured values, and the maximum value (Jmax) µg/cm²/hr was determined.

The evaluation results for each patch are shown below.
[Table 2]

**Table 2 Evaluation Results**

| | Tack | Peel | Skin permeation Jmax |
|---|---|---|---|
| Reference Ex. 1 | 287 | 76 | |
| Reference Ex. 2 | 238 | 316 | |
| Reference Ex. 3 | 243 | 159 | |
| Ex. 4 | 307 | 252 | 6.82 |
| Ex. 5 | 425 | 255 | 4.00 |
| Ex. 6 | 315 | 186 | 2.78 |
| Ex. 7 | 283 | 274 | 4.21 |
| Ex. 8 | 221 | 217 | 4.32 |
| Ex. 9 | 154 | 200 | 3.43 |
| Comp. Ex. 1 | 200 | Cohesive failure | |
| Comp. Ex. 2 | 193 | Cohesive failure | |
| Comp. Ex. 3 | 305 | Cohesive failure | |
| Comp. Ex. 4 | 293 | 88 | |
| Comp. Ex. 5 | 309 | Cohesive failure | 7.00 |
| Comp. Ex. 6 | 290 | Cohesive failure | 4.71 |
| Comp. Ex. 7 | 225 | Cohesive failure | 3.82 |
| Comp. Ex. 8 | 246 | Cohesive failure | |
| Comp. Ex. 9 | 208 | Cohesive failure | 3.16 |
| Comp. Ex. 10 | 338 | Cohesive failure | 3.32 |
| Comp. Ex. 11 | 430 | Cohesive failure | 2.38 |
| Comp. Ex. 12 | 366 | Cohesive failure | 1.97 |
| Comp. Ex. 13 | 473 | Cohesive failure | 3.75 |
| Comp. Ex. 14 | 343 | 125 | 2.78 |

The preparation for external use of the present invention did not show cohesive failure, had excellent cohesive strength and appropriate pressure-sensitive adhesion, and a high percutaneous drug absorption was exhibited.

Patches were prepared in the same manner as in Example 4 at the mixing proportions below using tamsulosin free base instead of tamsulosin hydrochloride.
[Table 3]

**Table 3 Mixing proportions**

| | Tamsulosin (free form) | Duro-Tak 87-2287 | SMO | SML | Sodium acetate | Acetic acid | Aerosil | Boric acid | Coating thickness g/m² |
|---|---|---|---|---|---|---|---|---|---|
| Ex. 10 | 5 | Remainder | 4.5 | 3 | 1.51 | 1.47 | 6 | 5 | 110 |
| Ex. 11 | 5 | Remainder | 8.5 | 3 | 1.51 | 1.47 | 6 | 5 | 110 |
| Ex. 12 | 5 | Remainder | 12.2 | 3 | 1.51 | 1.47 | 6 | 5 | 110 |
| Ex. 13 | 5 | Remainder | 6.0 | 3 | 0 | 0 | 6 | 5 | 100 |
| Ex. 14 | 5 | Remainder | 10.0 | 3 | 0 | 0 | 2 | 5 | 110 |
| Ex. 15 | 5 | Remainder | 10.0 | 3 | 0 | 0 | 3 | 5 | 110 |
| Ex. 16 | 5 | Remainder | 10.0 | 3 | 0 | 0 | 4 | 5 | 110 |
| Ex. 17 | 5 | Remainder | 10.0 | 3 | 0 | 0 | 5 | 5 | 110 |
| Ex. 18 | 5 | Remainder | 10.0 | 3 | 0 | 0 | 6 | 5 | 110 |

| | | | | | | | | | |
|---|---|---|---|---|---|---|---|---|---|
| SMO: sorbitan monooleate | | | | | | | | | |

Results obtained by evaluating the patches thus prepared in the same manner are given below.
[Table 4]

**Table 4 Evaluation results**

| | Tack | Peel | Skin permeation Jmax |
|---|---|---|---|
| Ex. 10 | 336 | 630 | 8.38 |
| Ex. 11 | 294 | 676 | 8.99 |
| Ex. 12 | 30 | 770 | 10.1 |
| Ex. 13 | 148 | 198 | 4.32 |
| Ex. 14 | 226 | | 5.83 |
| Ex. 15 | 207 | | 6.32 |
| Ex. 16 | 225 | | 5.61 |
| Ex. 17 | 206 | | 5.26 |
| Ex. 18 | 188 | 156 | 5.41 |

The preparation for external use of the present invention did not show cohesive failure in the case of tamsulosin, and had excellent percutaneous absorption.

Patches having the mixing proportions below were prepared in the same manner as in Example 4 except that the type of absorption enhancer and the amount thereof added were changed.
[Table 5]

**Table 5**

| | Tamsulosin hydrochloride | Duro-Tak 87-2287 | IPM | SML | SMO | LAD A | Capric acid | Sodium acetate | Aerosil | Boric acid | Coating thickness (g/m²) |
|---|---|---|---|---|---|---|---|---|---|---|---|
| Ex. 19 | 5 | Remainder | 5 | 3 | | | | 1.4 | 6 | 5 | 100 |
| Ex. 20 | 5 | Remainder | 5 | | | | | 2.8 | 6 | 5 | 110 |
| Ex. 21 | 5 | Remainder | | 5 | | | | 2.8 | 6 | 5 | 110 |
| Ex. 22 | 5 | Remainder | | | | 5 | | 2.8 | 6 | 5 | 110 |
| Ex. 23 | 5 | Remainder | | | | | | 2.8 | 6 | 5 | 100 |
| Ex. 24 | 5 | Remainder | | | | | 5 | 2.8 | 6 | 5 | 120 |
| Ex. 25 | 5 | Remainder | | | | 5 | 5 | 2.8 | 6 | 5 | 120 |
| Ex. 26 | 5 | Remainder | | 5 | | | 5 | 2.8 | 6 | 5 | 120 |
| Ex. 27 | 5 | Remainder | | 5 | | 5 | | 2.8 | 6 | 5 | 110 |
| Ex. 28 | 5 | Remainder | | | | 1 | 1 | 2.8 | 6 | 5 | 110 |
| Ex. 29 | 5 | Remainder | | | | 3 | 1 | 2.8 | 6 | 5 | 110 |
| Ex. 30 | 5 | Remainder | | | | 2. 5 | 2.5 | 2.8 | 6 | 5 | 110 |
| Ex. 31 | 5 | Remainder | | 1 | | 1 | | 2.8 | 6 | 5 | 110 |
| Ex. 32 | 5 | Remainder | | 3 | | 1 | | 2.8 | 6 | 5 | 110 |
| Ex. 33 | 5 | Remainder | | 5 | | 1 | | 2.8 | 6 | 5 | 110 |
| Ex. 34 | 5 | Remainder | | 1 | | 3 | | 2.8 | 6 | 5 | 110 |
| Ex. 35 | 5 | Remainder | | 3 | | 3 | | 2.8 | 6 | 5 | 110 |
| Ex. 36 | 5 | Remainder | | 5 | | 3 | | 2.8 | 6 | 5 | 110 |
| Ex. 37 | 5 | Remainder | | 1 | | 5 | | 2.8 | 6 | 5 | 110 |
| Ex. 38 | 5 | Remainder | | 3 | | 5 | | 2.8 | 6 | 5 | 110 |
| Ex. 39 | 5 | Remainder | | 5 | | 5 | | 2.8 | 6 | 5 | 110 |
| Ex. 40 | 5 | Remainder | 3 | 5 | | 5 | | 2.8 | 6 | 5 | 110 |
| Ex. 41 | 5 | Remainder | 5 | 5 | | 5 | | 2.8 | 6 | 5 | 110 |
| Ex. 42 | 5 | Remainder | | 5 | 1 | 3 | | 2.8 | 6 | 5 | 110 |
| Ex. 43 | 5 | Remainder | | 5 | 3 | 3 | | 2.8 | 6 | 5 | 110 |
| Ex. 44 | 5 | Remainder | | 5 | 5 | 3 | | 2.8 | 6 | 5 | 110 |

| | | | | | | | | | | | |
|---|---|---|---|---|---|---|---|---|---|---|---|
| LADA: Lauric acid diethanolamide | | | | | | | | | | | |

Results obtained by evaluating the patches thus prepared in the same manner are given below.
[Table 6]

**Table 6 Evaluation results**

| | Tack | Peel | Skin permeation Jmax (hairless mouse) | Skin permeation Jmax (human) |
|---|---|---|---|---|
| Ex. 19 | 291 | 158 | 7.29 | |
| Ex. 20 | 358 | 159 | 5.18 | |
| Ex. 21 | 341 | 180 | 8.07 | |
| Ex. 22 | 286 | 151 | 8.68 | |
| Ex. 23 | 215 | 149 | 3.46 | |
| Ex. 24 | 165 | 201 | 6.54 | |
| Ex. 25 | 216 | 208 | 40.78 | 0.36 |
| Ex. 26 | 280 | 195 | 12.28 | |
| Ex. 27 | 287 | 209 | 10.30 | |
| Ex. 28 | 297 | 202 | 9.66 | |
| Ex. 29 | 264 | 201 | 18.35 | 0.59 |
| Ex. 30 | 196 | 180 | 15.76 | |
| Ex. 31 | 352 | 190 | 6.71 | |
| Ex. 32 | 349 | 295 | 7.58 | |
| Ex. 33 | 346 | 322 | 10.25 | |
| Ex. 34 | 293 | 303 | 7.06 | |
| Ex. 35 | 307 | 301 | 10.40 | |
| Ex. 36 | 318 | 321 | 11.75 | |
| Ex. 37 | 334 | 313 | 10.64 | |
| Ex. 38 | 299 | 330 | 14.53 | |
| Ex. 39 | 290 | 320 | 16.77 | |
| Ex. 40 | 354 | 314 | 13.11 | |
| Ex. 41 | 391 | 362 | 13.95 | |
| Ex. 42 | 248 | 301 | 9.34 | |
| Ex. 43 | 333 | 403 | 11.12 | |
| Ex. 44 | 302 | 410 | 13.57 | |

The preparation for external use of the present invention did not show cohesive failure, and had excellent percutaneous absorption.

### Test 2

Actual residue from the preparation for external use of the present invention was examined.

### Test Method

The back of Japanese white rabbits was clipped and shaved, and 4 cm² preparations of Example 3, Example 7, Comparative Example 4, and Comparative Example 14 were administered.

Jackets were put on the rabbits, the preparations were peeled off after 72 hours, and the residue was examined.
Animal species: Jw (Japanese white rabbit) female
Age: 19 to 20 weeks
N = 4
The results showed that there was no residue for Example 3 and Example 7, and there was residue (cohesive failure) for Comparative Example 4 and Comparative Example 14.
It was thus shown that the patch of the present invention did not show cohesive failure when used in practice, whereas in the case of one containing only 5% of boric acid, when it was actually administered to the skin (rabbit) for 3 days there was residue (cohesive failure).

The concentrations of tamsulosin hydrochloride in plasma when the patch of Example 41 of the present invention is administered by being continuously administered and when there is a drug suspension period were compared with the plasma concentration when a commercial product 1 and a commercial product 2, which are preparations of tamsulosin hydrochloride, are administered orally once a day. FIG. 1 shows the results. Comparing the administration once a day continuously of Example 41 with the commercial products, the patch of the present invention showed less change in blood drug concentration, that is, the P/T ratio was small. Furthermore, it can be seen that the patch of the present invention showed less change in blood drug concentration even when a drug suspension period was provided with one day administered and one day suspension of the drug as in (2), while maintaining a certain level of drug concentration.

### Industrial Applicability

The preparation for external use of the present invention is used in the pharmaceutical industry for the purpose of preventing or treating a disease. Furthermore, the preparation for external use containing tamsulosin or an acid addition salt thereof is used in the pharmaceutical industry for the purpose of improving dysuria accompanying prostatic enlargement.

## Claims

1. A preparation for external use comprising a pressure-sensitive adhesive matrix layer, the pressure-sensitive adhesive matrix layer comprising tamsulosin and/or a pharmaceutically acceptable acid addition salt thereof, a boron compound, a silicic acid, and a pressure-sensitive adhesive base comprising a hydroxy group-containing polymer.

2. The preparation for external use according to Claim 1, wherein it has a backing.

3. The preparation for external use according to Claim 1 or 2, wherein the boron compound is boric acid.

4. The preparation for external use according to any one of Claims 1 to 3, wherein the silicic acid is hydrophilic anhydrous silicic acid.

5. The preparation for external use according to any one of Claims 1 to 4, wherein it further comprises an absorption enhancer and/or a plasticizer.

6. The preparation for external use according to Claim 5, wherein the absorption enhancer is one or more selected from the group consisting of a fatty acid, a fatty acid salt, a fatty acid ester, and a fatty acid amide.

7. The preparation for external use according to Claim 6, wherein the absorption enhancer is one or more selected from the group consisting of acetic acid, capric acid, sodium acetate, isopropyl myristate, sorbitan monooleate, sorbitan monolaurate, and lauric acid diethanolamide.

8. The preparation for external use according to any one of claims 1-7, wherein the amount of tamsulosin and/or a pharmaceutically acceptable acid addition salt thereof is in the range of 1 to 30 mass % relative to the entire pressure-sensitive adhesive matrix layer.

9. The preparation for external use according to any one of claims 1-8, wherein the amount of the boron compound is in the range of 1 to 8 mass % relative to the entire pressure-sensitive adhesive matrix layer.

10. The preparation for external use according to any one of claims 1-9, wherein the amount of the silicic acid is in the range of 0.2 to 10 mass % relative to the entire pressure-sensitive adhesive matrix layer.

11. The preparation for external use according to any one of claims 1-10, wherein the proportion of the hydroxy group-containing polymer is at least 25 mass % relative to the entire pressure-sensitive adhesive matrix layer.

## Patentansprüche

1. Zubereitung zur äußeren Anwendung umfassend eine Haftklebermatrixschicht, wobei die Haftklebermatrixschicht Folgendes enthält Tamsulosin und/oder ein pharmazeutisch annehmbares Säureadditionssalz davon, eine Borverbindung, eine Kieselsäure und eine Haftkleberbasis, die ein Hydroxylgruppen-haltiges Polymer umfasst.

2. Zubereitung zur äußeren Anwendung gemäß Anspruch 1, wobei diese eine Unterlage aufweist.

3. Zubereitung zur äußeren Anwendung gemäß Anspruch 1 oder 2, wobei die Borverbindung Borsäure ist

4. Zubereitung zur äußeren Anwendung gemäß einem der Ansprüche 1 bis 3, wobei die Kieselsäure hydrophile wasserfreie Kieselsäure ist.

5. Zubereitung zur äußeren Anwendung gemäß einem der Ansprüche 1 bis 4, wobei diese weiterhin einen Absorptionsverstärker und/oder einen Weichmacher aufweist

6. Zubereitung zur äußeren Anwendung gemäß Anspruch 5, wobei der Absorptionsverstärker ein Absorptionsverstärker ist oder mehrere Absorptionsverstärker sind, ausgewählt aus der Gruppe, bestehend aus einer Fettsäure, einem Fettsäuresalz, einem Fettsäureester und einem Fettsäureamid.

7. Zubereitung zur äußeren Anwendung gemäß Anspruch 6, wobei der Absorptionsverstärker ein Absorptionsverstärker ist oder mehrere Absorptionsverstärker sind, ausgewählt aus der Gruppe, bestehend aus Essigsäure, Caprinsäure, Natriumacetat, Isopropylmyristat, Sorbitanmonooleat, Sorbitanmonolaurat und Laurinsäurediethanolamid.

8. Zubereitung zur äußeren Anwendung gemäß einem der Ansprüche 1 bis 7, wobei die Menge an Tamsulosin und einem pharmazeutisch annehmbaren Säureadditionssalzes davon im Bereich von 1 bis 30 Massenprozent liegt, bezogen auf die gesamte Haftklebermatrixschicht.

9. Zubereitung zur äußeren Anwendung gemäß einem der Ansprüche 1 bis 8, wobei die Menge an der Borverbindung im Bereich von 1 bis 8 Massenprozent liegt, bezogen auf die gesamte Haftklebermatrixschicht.

10. Zubereitung zur äußeren Anwendung gemäß einem der Ansprüche 1 bis 9, wobei die Menge an der Kieselsäure im Bereich von 0,2 bis 10 Massenprozent liegt, bezogen auf die gesamte Haftklebermatrixschicht.

11. Zubereitung zur äußeren Anwendung gemäß einem der Ansprüche 1 bis 10, wobei der Anteil des Hydroxylgruppen-haltigen Polymers wenigstens 25 Massenprozent beträgt, bezogen auf die gesamte Haftklebermatrixschicht.

## Revendications

1. Une préparation à usage externe comprenant une couche de matrice adhésive sensible à la pression, la couche de matrice adhésive sensible à la pression comprenant de la tamsulosine et/ou un sel d'addition acide de celle-ci acceptable du point de vue pharmaceutique, un composé du bore, un acide silicique, et une base adhésive sensible à la pression comprenant un polymère qui contient un groupe hydroxy.

2. La préparation à usage externe selon la revendication 1, ladite préparation possédant un support.

3. La préparation à usage externe selon la revendication 1 ou 2, le composé du bore étant l'acide borique.

4. La préparation à usage externe selon l'une quelconque des revendications 1 à 3, l'acide silicique étant un acide silicique anhydre hydrophile.

5. La préparation à usage externe selon l'une quelconque des revendications 1 à 4, ladite préparation comprenant en outre un agent favorisant l'absorption et/ou un plastifiant.

6. La préparation à usage externe selon la revendication 5, l'agent favorisant l'absorption étant un ou plusieurs composés sélectionnés dans le groupe constitué d'un acide gras, d'un sel d'acide gras, d'un ester d'acide gras, et d'un amide d'acide gras.

7. La préparation à usage externe selon la revendication 6, l'agent favorisant l'absorption étant un ou plusieurs composés sélectionnés dans le groupe constitué de l'acide acétique, de l'acide caprique, de l'acétate de sodium, du myristate d'isopropyle, du monooléate de sorbitan, du monolaurate de sorbitan, et du diéthanolamide de l'acide laurique.

8. La préparation à usage externe selon l'une quelconque des revendications 1 à 7, la quantité de tamsulosine et/ou d'un sel d'addition acide de celle-ci acceptable du point de vue pharmaceutique étant dans la gamme de 1 à 30% en masse par rapport à toute la couche de matrice adhésive sensible à la pression.

9. La préparation à usage externe selon l'une quelconque des revendications 1 à 8, la quantité du composé du bore étant dans la gamme de 1 à 8% en masse par rapport à toute la couche de matrice adhésive sensible à la pression.

10. La préparation à usage externe selon l'une quelconque des revendications 1 à 9, la quantité d'acide silicique étant dans la gamme de 0,2 à 10% en masse par rapport à toute la couche de matrice adhésive sensible à la pression.

11. La préparation à usage externe selon l'une quelconque des revendications 1 à 10, la proportion du polymère qui contient un groupe hydroxy étant d'au moins 25% en masse par rapport à toute la couche de matrice adhésive sensible à la pression.
